(19) Europäisches Patentamt
European Patent Office
Office européen des brevets

(11) Veröffentlichungsnummer: **0 216 884**
**B1**

(12) **EUROPÄISCHE PATENTSCHRIFT**

(45) Veröffentlichungstag der Patentschrift: **08.08.90**

(21) Anmeldenummer: **86902384.6**

(22) Anmeldetag: **27.03.86**

(86) Internationale Anmeldenummer:
**PCT/EP86/00183**

(87) Internationale Veröffentlichungsnummer:
**WO 86/05777 09.10.86 Gazette 86/22**

(51) Int. Cl.$^5$: **C 07 C 49/84**, C 07 C 49/83, C 07 C 49/82, C 07 C 69/00, C 07 C 59/90, C 07 C 59/84, C 07 C 309/04, C 07 C 309/07, C 07 C 225/16, C 07 C 215/04

(54) **FOTOINITIATOREN FÜR DIE FOTOPOLYMERISATION VON UNGESÄTTIGTEN SYSTEMEN.**

(30) Priorität: **03.04.85 DE 3512179**

(43) Veröffentlichungstag der Anmeldung:
**08.04.87 Patentblatt 87/15**

(45) Bekanntmachung des Hinweises auf die Patenterteilung:
**08.08.90 Patentblatt 90/32**

(84) Benannte Vertragsstaaten:
**AT BE CH DE FR GB IT LI NL SE**

(56) Entgegenhaltungen:
**CH-A- 401 943**
**DE-A-2 000 365**
**DE-A-2 001 288**
**DE-A-2 232 365**
**DE-A-2 730 462**
**FR-A-1 059 911**
**FR-A-1 505 328**
**FR-A-1 516 775**
**FR-A-2 278 327**
**FR-A-2 300 552**
**FR-A-2 391 183**
**FR-A-2 427 329**
**NL-A-6 918 625**
**US-A-2 182 786**
**US-A-3 914 286**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **KÖHLER, Manfred,Dr.**
**Hebbelstrasse 38**
**D-6100 Darmstadt (DE)**
Erfinder: **OHNGEMACH, Jörg,Dr.**
**Taubenstrasse 6**
**D-6107 Reinheim (DE)**
Erfinder: **WEHNER, Gregor,Dr.**
**Reuterallee 12**
**D-6100 Darmstadt (DE)**
Erfinder: **GEHLHAUS, Jürgen,Dr.**
**Vier Morgen 7**
**D-6147 Lautertal 1 (DE)**

**EP 0 216 884 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Ketonderivate sowie deren Verwendung als Fotoinitiatoren für die Fotopolymerisation ethylenisch ungesättigter Verbindungen bzw. solche enthaltender Systeme.

Insbesondere betrifft die Erfindung die Verwendung dieser Verbindungen als Fotoinitiatoren für die Fotopolymerisation von ethylenisch ungesättigen Verbindungen in wäßrigen Systemen sowie wäßrige fotopolymerisierbare Systeme, enthaltend solche Ketonderivate. Des weiteren betrifft die Erfindung Fotopolymerisationsverfahren zur Herstellung von wäßrigen Polymerisat-Lösungen oder Polymerisat-Dispersionen sowie zur Herstellung wasserlöslicher oder hydrophiler Polymerisate, bei denen derartige Ketone als Fotoinitiatoren eingesetzt werden. Darüberhinaus betrifft die Erfindung die Verwendung dieser Verbindungen generell als Fotoinitiatoren, also auch in nichtwäßrigen Systemen, sowie entsprechende fotopolymerisierbare Systeme.

Fotochemisch induzierte Polymerisationsreaktionen haben in der Technik große Bedeutung erlangt, insbesondere wenn es um eine schnelle Härtung von dünnen Schichten geht, wie z.B. bei der Härtung von Lack- und Harzüberzügen auf Papier, Metall und Kunststoff oder bei der Trocknung von Druckfarben, da diese Verfahren sich gegenüber konventionellen Methoden zum Bedrucken und Beschichten von Gegenständen durch Rohstoff- und Energieersparnis und eine geringere Umweltbelastung auszeichnen.

Aber auch die Herstellung von Polymermaterialien an sich durch Polymerisation entsprechender ungesättigter monomerer Ausgangsmaterialien erfolgt vielfach fotochemisch. Bedeutungsvoll hierbei sind neben den üblichen Verfahren der Lösungspolymerisation in organischen Lösungsmitteln Lösung- und Emulsionspolymerisationen in wäßrigen Systemen.

Da bei den genannten Reaktionen in der Regel keiner der Reaktionspartner in der Lage ist, die fotochemisch wirksame Strahlung in ausreichendem Maße zu absorbieren, müssen sogenannte Fotoinitiatoren zugesetzt werden, die an der gewünschten Reaktion nicht teilnehmen, aber in der Lage sind, eingestrahltes Licht oder UV-Strahlen zu absorbieren, die dabei aufgenommene Energie auf einen der Reaktionspartner zu übertragen und so aktive Starterradikale zu bilden, die ihrerseits die Fotopolymerisation auslösen. Wesentliche Kriterien für die Auswahl solcher Initiatoren sind unter anderem die Art der durchzuführenden Reaktionen, das Verhältnis des Absorptionsspektrums des Initiators zur spektralen Energieverteilung der verfügbaren Strahlenquelle, die Löslichkeit des Initiators in der Reaktionsmischung, die Dunkellagerstabilität des mit dem Initiator versetzten Reaktionssystems sowie die Beeinflussung der Endprodukte durch darin verbliebene Reste des Initiators und/oder der daraus während der fotochemischen Reaktion entstandenen Produkte. Insbesondere die Geschwindigkeit der Reaktion hängt stark vom verwendeten Initiator ab. Deshalb hat es nicht an Versuchen gefehlt, neue Initiatoren zu suchen, die in ihrem Vermögen, die Fotopolymerisation ethylenisch ungesättigter Verbindungen bzw. die Härtung fotopolymerisierbare Systeme zu initiieren, eine gesteigerte Reaktivität zeigen.

Als Initiatoren für die Fotopolymerisation ungesättigter Verbindungen sind bisher hauptsächlich Benzophenonderivate, Benzoinether, Benzilketale, Dibenzosuberonderivate, Anthrachinone, Xanthone, Thioxanthone, α-Halogenacetophenonderivate, Dialkoxyacetophenone und Hydroxyalkylphenone eingesetzt worden.

Die technische Anwendbarkeit vieler der genannten Substanzen wird jedoch bekanntermaßen durch eine Reihe von Nachteilen zum Teil deutlich eingeschränkt. Hierzu gehört insbesondere die häufig zu geringe Reaktivität im Vermögen, die Fotopolymerisation ethylenisch ungesättigter Verbindungen zu initiieren. Neben molekülspezifischer Reaktivität spielt hierbei vielfach die Löslichkeit bzw. die möglichst gleichmäßige Einarbeitbarkeit der Fotoinitiatoren in den fotopolymerisierbaren Systemen eine entscheidende Rolle.

Für eine besondere Eignung für Fotopolymerisationsreaktionen in wäßrigen Systemen bedeutet dies, daß die Fotoinitiatoren im üblich angewendeten Konzentrationsbereich genügend wasserlöslich sein und gleichzeitig so reaktiv sein müssen, daß eine wirtschaftliche Ausnutzung der Bestrahlungsapparaturen gewährleistet ist. Wirtschaftliche Ausnutzung bedeutet hierbei nicht nur die für eine vollständige Umwandlung von Monomermaterial in Polymerisat erforderliche Energiemenge, die sich etwa in der bereitzustellenden Lampenleistung und der Bestrahlungsdauer niederschlägt, sondern vielfach auch der unter den Prozeßbedingungen erzielbare Polymerisationsgrad des herzustellenden Polymers, da hiervon vielfältige Produkteigenschaften und entsprechende Anwendungsmöglichkeiten abhängen.

Fotopolymerisationen in wäßrigen Systemen mit Hilfe ausgewählter gängiger Fotoinitiatoren sind bekannt. Beispielsweise wird in der deutschen Offenlegungsschrift DE-OS 2354006 ein Verfahren zur Herstellung stabiler Wasser-in-Öl-Emulsionen von wasserlöslichen Polymerisaten beschrieben, wobei wasserlösliche Monomere in eine Wasser-in-Öl-Emulsion eingebracht werden und unter Zusatz mindestens eines Fotoinitiators fotopolymerisiert werden. Die deutsche Offenlegungsschrift DE-OS 2831263 beschreibt die Herstellung von wäßrigen Polymerlösungen und Polymerdispersionen durch Fotopolymerisation, wobei Benzoinderivate, die quartäre Ammoniumgruppen enthalten, als Fotoinitiatoren eingesetzt werden. In den europäischen Patentanmeldungen EP 0047009 und EP 0068189 wird die Herstellung hydrophiler Polymermaterialien durch fotochemische Lösungs- bzw. Emulsionspolymerisation im wäßrigen Medium beschrieben, wobei auch Fotoinitiatoren eingesetzt werden.

Die in diesem Zusammenhang bekannt gewordenen Fotoinitiatoren genügen jedoch nicht den heutigen Ansprüchen hinsichtlich Reaktivität und Wasserlöslichkeit.

Aus DE-OS 2722264 sind hochreaktive Fotoinitiatoren vom Hydroxyalkylphenon-Typ bekannt. Diese sind aufgrund ihres hydrophoben Charakters in wässrigen Bindemittelsystemen nur bedingt einsetzbar.

Es bestand somit die Aufgabe, Fotoinitiatoren aufzufinden, die sowohl eine vorzügliche Löslichkeit in wäßrigen Systemen als auch eine besonders hohe Reaktivität, insbesondere im Hinblick auf die Erzielung besonders hoher Molmassen, respektive polymerisationsgrade, in den herzustellenden Polymerisaten aufweisen.

Es wurde nun gefunden, daß diese Forderungen in hervorragender Weise von Ketonderivaten der allgemeine Formel I,

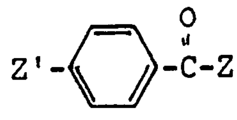

(I)

worin

Z $CR^1R^2(OR^3)$ bedeutet mit
$R^1$ H, $C_{1-6}$-Alkyl oder Phenyl,
$R^2$ H, $C_{1-6}$-Alkyl oder $C_{1-6}$-Alkoxy,
$R^3$ H, $C_{1-6}$-Alkyl oder $C_{1-6}$-Alkanoyl

und

Z' $Y\text{-}[(CH_2)_m\text{-}X]_n$-bedeutet mit
X $CH_2$ oder O,
y OH; COOH, $SO_3H$ einschließlich deren Alkali-, Erdalkali- oder Ammoniumsalze sowie deren Salze organischer Stickstoffbasen; NRR' mit R und R' jeweils H, $C_{1-20}$-Alkyl oder $C_{1-4}$-Hydroxyalkyl, gegebenenfalls quarterniert oder in Form der Säureadditionssalze,
n, m jeweils die Zahlen 1-4,

erfüllt werden.

Die Verbindungen der allgemeinen Formel I sind hochwirksame Fotoinitiatoren und können generell in fotopolymerisierbaren Systemen eingesetzt werden, sofern in diesen ethylenisch ungesättigte fotopolymerisierbare Verbindungen enthalten sind.

Die erfindungsgemäßen Fotoinitiatoren besitzen darüberhinaus, bedingt durch den speziellen Substituenten Z' am Phenylring der Phenon-Struktureinheit, einen ausgeprägt hydrophilen bzw. grenzflächenaktiven Charakter, der diesen Verbindungen eine hohe Wasserlöslichkeit und somit vorzügliche Eigenschaften hinsichtlich einer gleichmäßigen Verteilbarkeit in wäßrigen fotopolymerisierbaren Systemen verleiht. Dies kommt besonders vorteilhaft sowohl in solchen Systemen zum Ausdruck, in denen sich das Monomermaterial in wäßriger Lösung befindet, als auch in Systemen, in denen die fotopolymerisierbaren Komponenten in wäßrigem Medium dispergiert vorliegen.

Gegenstand der Erfindung sind somit die Verbindungen der allgemeinen Formel I.

Gegenstand der Erfindung ist außerdem die Verwendung von Ketonderivaten der allgemeinen Formel I als Fotoinitiatoren für die Fotopolymerisation von ethylenisch ungesättigten Verbindungen oder solche enthaltender Systeme. Gegenstand der Erfindung ist insbesondere die Verwendung der Verbindungen der Formel I als Fotoinitiatoren in wäßrigen Systemen, etwa bei der Herstellung wäßriger Polymerisat-Lösungen oder Polymerisat-Dispersionen oder bei der Strahlungshärtung von Beschichtungen auf Basis wäßriger Polymerisat-Dispersionen.

Gegenstand der Erfindung sind weiterhin wäßrige und nichtwäßrige fotopolymerisierbare Systeme, enthaltend mindestens eine ethylenisch ungesättigte fotopolymerisierbare Verbindung sowie gegebenenfalls weitere bekannte und übliche Zusatzstoffe, wobei diese Systeme mindestens eine der Verbindungen der Formel I als Fotoinitiator enthält sowie Verfahren zu deren Fotopolymerisation.

Gegenstand der Erfindung sind darüber hinaus Fotopolymerisationsverfahren zur Herstellung von wäßrigen Polymerisat-Lösungen oder Polymerisat-Dispersionen sowie zur Herstellung wasserlöslicher oder hydrophiler Polymerisate, wobei die Verbindungen der Formel I als Fotoinitiatoren eingesetzt werden.

Die erfindungsgemäßen Ketonderivate leiten sich hinsichtlich ihrer strahlungsreaktiven Wirkgruppen strukturell von bekannten Fotoinitiatorstrukturen ab. In der allgemeinen Formel I kann Z die Gruppierung $\text{-}CR^1R^2(OR^3)$ oder Phenyl bedeuten. Im ersten Fall ergeben sich Ketonderivate der Unterformel Ia

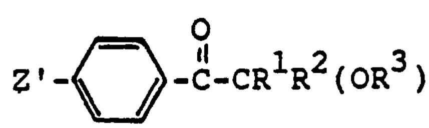

(Ia)

worin Z', $R^1$, $R^2$ und $R^3$ die oben angegebenen Bedeutungen haben. Stehen $R^1$ und $R^2$ jeweils für Wasserstoff oder Alkyl und bedeutet $R^3$ Wasserstoff, Alkyl oder Alkanoyl, so ergeben sich aus Unterformel

Ia durch Z' substituierte Hydroxy-, Alkoxy-und Alkanoyloxyalkylphenone, stellen somit Derivate dieser mittlerweile sehr bedeutungsvollen Klasse von Fotoinitiatoren dar. Bedeutet in Unterformel Ia $R^1$ Alkyl oder Phenyl und $R^2$ Alkoxy, so gehören die entsprechenden Verbindungen zur Klasse der Benzilketale und Dialkoxyacetophenone. Ist $R^1$ Phenyl und $R^2$ Wasserstoff oder Alkyl, so sind die entsprechenden Verbindungen Benzoine bzw. Benzoinetherderivate.

Bevorzugte Fotoinitiatoren gemäß der Erfindung sind Verbindungen nach Unterformel Ia von denen wiederum die entsprechenden Hydroxyalkylphenonderivate besonders bevorzugt sind. In diesen Strukturen kann $R^1$ und $R^2$ geradoder verzweigtkettiges Alkyl mit bis zu 6 C-Atomen sein. Bevorzugte Hydroxyalkylphenonderivate sind solche, in denen $R^1$ und $R^2$ Methyl ist. $R^1$ und $R^2$ können aber zusammen auch ein Cycloalkansystem bilden. Verbindungen in denen diese einen Cyclohexanring bilden, sind ebenfalls bevorzugt.

In Formel I steht Z' für die Gruppierung $Y-[(CH_2)_m-X]_n-$. X kann hierin $CH_2$ oder O bedeuten; n und m stehen jeweils für die Zahlen 1-4.

Ist X $CH_2$, so ergeben sich Verbindungen, in denen der Rest Y über eine Alkylenbrücke mit 2-20 C-Atomen mit dem Phenylring der Phenon-Struktureinheit verknüpft ist, entsprechend der Unterformel Ic,

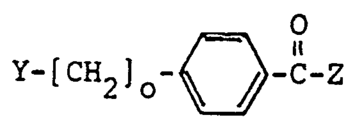

(Ic)

worin o die Zahlen 2-20 repräsentiert.

Steht X für O, so ergeben sich Verbindungen in denen der Rest Y über 1-4 Alkenyloxygruppen mit dem Phenylring verknüpft ist, entsprechend Unterformel Id

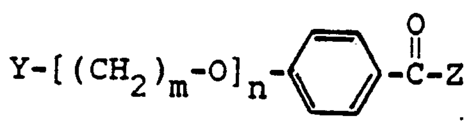

(Id)

Verbindungen gemäß Formel Ic, in denen o die Zahlen 2-5 darstellt, sowie gemäß Formel Id, in denen n und m jeweils 1 oder 2 bedeuten, sind bevorzugt.

Y steht für OH, COOH, $SO_3H$ sowie NRR'. Entsprechende Carbon- bzw. Sulfonsäure-Derivate können auch in Form ihrer Alkali-, Erdalkali- oder Ammoniumsalze sowie als Salze organischer Stickstoffbasen vorliegen. Letztere können sein organische Amine, in denen der Aminostickstoff eine oder mehrere Alkylgruppen mit bis zu 20 C-Atomen und/oder Hydroxyalkylgruppen mit bis zu 4 C-Atomen tragen kann oder in denen der Stickstoff Teil eines heterozyklischen Systems ist. Dies können sein insbesondere tertiäre Alkyl- bzw. Hydroxyalkylamine, Pyridin-, Piperidin- und Morpholinderivate. Besonders bevorzugte Salze mit organischen Stickstoffbasen sind solche, in denen diese Basen organische Amine sind, die als Koinitiatoren für Fotoinitiatoren bekannt sind oder die Tensidcharakter aufweisen. Beispiele hierfür sind Methyldiethanolamin, N-(2-Hydroxyethyl)-morpholin, Cetylamin, Cetylpyridin.

In Verbindungen, in denen Y für NRR' steht kann R und R' jeweils Wasserstoff, Alkyl mit bis zu 20 C-Atomen oder Hydroxyalkyl mit bis zu 4 C-Atomen sein. Derartige Aminoderivate können aber auch quarterniert sein oder in Form der Säureadditionssalze vorliegen wie beispielsweise in Form ihrer Salzsäure-, Schwefelsäure- oder Toluolsulfonsäuresalze.

Kombiniert man jeweils die Strukturteile gemäß den Unterformeln 1a oder 1b mit jenen der Unterformeln 1c oder 1d, insbesondere die jeweils bevorzugten Strukturvarianten, so gelangt man zu den bevorzugten Verbindungen der allgemeinen Formel I.

Es sind dies beispielsweise:

4-(2-Hydroxyethyl)-phenyl-2-hydroxy-2-propylketon
4-(3-Hydroxypropyl)-phenyl-2-hydroxy-2-propylketon
4-(2-Aminoethyl)-phenyl-2-hydroxy-2-propylketon
4-(3-Aminopropyl)-phenyl-2-hydroxy-2-propylketon
4-(2-Hydroxycarbonylethyl)-phenyl-2-hydroxy-2-propylketon
4-(3-Hydroxycarbonylpropyl)-phenyl-2-hydroxy-2-propylketon
4-(2-Hydroxyethoxyl-phenyl-2-hydroxy-2-propylketon
4-(2-Aminoethoxy)-phenyl-2-hydroxy-2-propylketon
4-[2-(Hydroxymethoxy)-ethoxy]-phenyl-2-hydroxy-2-propylketon
4-(2-Hydroxydiethoxy)-phenyl-2-hydroxy-2-propylketon
4-[2-(Hydroxycarbonylmethoxy)-ethoxy]-phenyl-2-hydroxy-2-propylketon
4-(2-Hydroxyethoxy)-diethoxyacetophenon

Besonders bevorzugt ist die Verbindung:

4-(2-Hydroxyethoxy)-phenyl-2-hydroxy-2-propylketon

Die Verbindungen der allgemeinen Formel I können nach Standardverfahren der organischen Chemie hergestellt werden. Die Reaktionsbedingungen hierbei können den Standardwerken der präparativen

organischen Chemie entnommen werden, z. B. HOUBEN-WEYL, Methoden der organischen Chemie, Georg-Thieme Verlag, Stuttgart, oder ORGANIC SYNTHESIS, J. Wiley, New York London Sydney.

Verbindungen der Allgemeinen Formel I können etwa in der Weise erhalten werden, daß man geeignete Phenylderivate die durch Z' oder eine Gruppierung, die leicht in Z' überführbar ist, substituiert sind, zur Einführung der Fotoinitiatorwirkstruktur oder einer Vorstufe hiervon mit einem entsprechenden Carbonsäurehalogenid nach Friedel-Crafts acyliert.

Als Ausgangsstoffe geeignete Phenylderivate sind etwa Ω-Phenylalkanole wie 2-Phenylethanol, Phenyl- oder Phenoxyalkancarbonsäuren wie Dihydrozimtsäure oder Phenoxyessigsäure, ein- oder mehrfach ethoxyliertes Phenol wie 2-Hydroxyethylphenylether geeignet. Für die Friedel-Crafts-Acylierung empfiehlt es sich, die terminalen funktionellen Gruppen durch geeignete, später Wieder entfernbare Schutzgruppen zu schützen, etwa durch Acylierung im Falle der OH-Gruppe oder Veresterung im Falle der COOH-Gruppe.

Zur Erzeugung der Fotoinitiatorwirkstruktur des Hydroxyalkylphenontyps kann beispielsweise mit Isobuttersäurehalogenid oder α-Chlorisobuttersäurehalogenid acyliert und anschließend die Hydroxy-, Alkoxy- oder Alkanoyloxygruppierung eingeführt werden. So führt beispielsweise die Friedel-Crafts-Acylierung von acyliertem 2-Hydroxyethylphenylether mit Isobuttersäurechlorid und die anschließende Bromierung und Verseifung am tertiären C-Atom zu dem Fotoinitiator 4-(2-Hydroxyethoxy)-phenyl-2-Hydroxy-2-propylketon.

Diese Verbindung kann auch zu weiteren Fotoinitiatoren der Formel I umgewandelt werden, etwa durch Ethoxylierung an der terminalen OH-Gruppe oder durch Umsetzung mit Bromessigester und anschließende Verseifung, wobei sich die Verbindung 4-[2-(Hydroxycarbonylmethoxy)-ethoxy]-phenyl-2-hydroxy-2-propylketon erhalten läßt.

Der Austausch der OH-Gruppe durch Amino nach üblichen Methoden führt zu den entsprechenden Aminoderivaten, die sich bei Bedarf auch in Form der Säureadditionssalze erhalten lassen.

Acyliert man die als Ausgangssubstanzen einzusetzenden Phenylderivate beispielsweise mit Benzoylhalogenid, so erhält man sofort die entsprechenden Benzophenonderivate.

Acylierung mit Acetylhalogenid oder Phenylessigsäurehalogenid und anschließende Oxidation führt zu Acetoinen bzw. Benzoinen, die wiederum zu den Dialkoxyacetophenonen, Benzoinethern und Benzilketalen weiterverarbeitet werden können.

Die Verbindungen der Formel I können als Fotoinitiatoren generell in fotopolymerisierbaren Systemen eingesetzt werden, sofern in diesen ethylenisch ungesättigte, fotopolymerisierbare Verbindungen enthalten sind. Hierbei erfolgt ihre Anwendung völlig analog wie für bekannte Fotoinitiatoren üblich.

Der Zusatz zu den fotopolymerisierbaren Systemen in den üblichen Mengen von etwa 0,01-20 Gew.%, vorzugsweise 0,1-10 Gew.%, geschieht in der regel durch einfaches lösen und Einrühren, da die meisten der erfindungsgemäß zu verwendenden Fotoinitiatoren flüssig oder in den zu polymerisierenden Systemen zumindest gut löslich sind. Unter einem zu polymerisierenden System wird ein Gemisch von durch freie Radikale initiierbaren mono- oder polyfunktionellen ethylenisch ungesättigten Monomeren, Oligomeren, Prepolymeren, Polymeren oder Mischungen dieser Oligomeren, Prepolymeren und Polymeren mit ungesättigten Monomeren verstanden, das, falls erforderlich oder erwünscht, weitere Zusätze wie z.B. Antioxidantien, Lichtstabilisatoren, Farbstoffe, Pigmente, aber auch weitere bekannte Fotoinitiatoren sowie Reaktionsbeschleuniger enthalten kann.

Als ungesättigte Verbindungen kommen alle diejenigen infrage, deren C=C-Doppelbindungen durch z.B. Halogenatome, Carbonyl-, Cyano-, Carboxy-, Ester-, Amid-, Ether- oder Arylgruppen oder durch konjugierte weitere Doppeloder Dreifachbindungen aktiviert sind. Beispiele für solche Verbindungen sind Vinylchlorid, Vinylidenchlorid, Acrylnitril, Methacrylnitril, Acrylamid, Methacrylamid. Methyl-, Ethyl-, n- oder tert. Butyl-, Cyclohexyl-, 2-Ethylhexyl-, Benzyl-, Phenyloxyethyl-, Hydroxyethyl-, Hydroxypropyl-, niederes Alkoxyethyl-, Tetrahydrofurfurylacrylat oder methacrylat, Vinylacetat, -propionat, -acrylat, -succinat, n-Vinylpyrrolidon, n-Vinylcarbazol, Styrol, Divinylbenzol, substituierte Styrole, sowie die Mischungen von solchen ungesättigten Verbindungen.

Die erfindungsgemäßen Verbindungen der Formel I werden bevorzugt verwendet als Fotoinitiatoren bei der UV-Härtung von dünnen schichten wie beispielsweise Lackbeschichtungen auf allen hierfür üblichen Materialien und trägern. Diese können vornehmlich Papier, Holz, textile Träger, Kunststoff und Metall sein. Ein wichtiges Anwendungsgebiet ist auch die Trocknung bzw. Härtung von Lacken, Druckfarben und Siebdruckmaterialien, von Denen letztere bevorzugt bei der Oberflächenbeschichtung bzw. -gestaltung von beispielsweise Dosen, Tuben und metallenen Verschlußkappen eingesetzt werden.

Erfindungsgemäß eignen sich die erfindungsgemäßen Verbindungen insbesondere vorzüglich als Fotoinitiatoren für die Fotopolymerisation ethylenisch ungesättigter Verbindungen in wäßrigen Systemen aufgrund ihres ausgeprägten hydrophilen Charakters.

Unter wäßrigen Systemen sind solche zu verstehen, in denen ethylenisch ungesättigte Monomermaterialien in Wasser gelöst und/oder emulgiert sind. Hierbei können diese Systeme entweder nur ein bestimmtes Monomer oder auch Gemische von copolymerisierbaren Monomeren unterschiedlichen Typs enthalten. Es kommen alle solche ethylenisch ungesättigten Verbindungen in Frage, die in ausreichendem Maße wasserlöslich sind oder sich problemlos in wasser emulgieren lassen. In erster linie trifft dies auf Vinyl- und Acrylverbindungen zu, die durch entsprechende funktionelle Gruppen eine hohe Polarität aufweisen. Beispiele hierfür sind vornehmlich olefinisch ungesättigte Mono- oder

Dicarbonsäuren, wie etwa Acrylsäure, Methacrylsäure, Malein- und Fumarsäure, deren Alkali- und Ammoniumsalze, Amide und Nitrile dieser Säuren, wie Acrylamid, Methacrylamid, Maleinimid, Acrylnitril, Methacrylnitril, wasserlösliche Vinylverbindungen, wie N-Vinylpyrrolidon und N-Vinylcarbazol. Die fotopolymerisierbaren wäßrigen Systeme können 10-80 Gew. %, vornehmlich 30-70 Gew. %, an diesen Monomermaterialien enthalten.

Neben den genannten Monomermaterialien können die strahlungshärtbaren Systeme noch weitere übliche copolymerisierbare ungesättigte Verbindungen enthalten. Dies können sein praktisch alle übrigen bisher nicht genannten Monomere wie Acryl- und Methacrylsäureester, Vinylderivate wie Vinylchlorid, Vinylidenchlorid, Styrol, Divinylbenzol, mehrfach ungesättigte Verbindungen als Quervernetzer wie etwa Ethylendiacrylat, Hexandioldiacrylat, Trimethyloldiacrylat oder Pentaerythrittriacrylat. Auch ist der Zusatz von ungesättigten Oligomeren, Präpolymeren oder Polymeren wie etwa auf Basis acrylierter Polyester-, Epoxy- und Urethanharze möglich. In wäßrigen Systemen liegen derartige copolymerisierbare Zusätze im allgemeinen gegenüber den wasserlöslichen Monomeren in untergeordneter menge vor und übersteigen in der Regel kaum einen Anteil von 20 Gew. % des gesamten polymerisierbaren Materials.

Im Falle von wäßrigen Systemen können diesen weiterhin noch übliche Cosolventien, Lösungsvermittler oder Emulgatoren zugefügt werden. Beispiele hierfür sind aliphatische Mono- oder Polyalkohole wie Methanol, Ethanol, Isopropanol, Glycol, Glycerin, anionische, kationische, amphotere oder nichtionische Tenside wie sulfonierte Paraffinkohlenwasserstoffe, Alkylsulfate, Alkalisalze von Fettsäuren, Fettalkoholsulfate, Polyglycolether, ethoxylierte Alkylphenole. Derartige Zusätze können in den allgemein üblichen Mengen eingesetzt werden.

Die Zubereitung von wäßrigen fotopolymerisierbaren Systemen erfolgt in an sich üblicher Weise, etwa durch Lösen bzw. homogenes Vermischen der einzelnen Komponenten, wobei die erfindungsgemäßen Fotoinitiatoren in den hierfür üblichen Konzentrationen von 0,01 bis etwa 20 Gew. %, bezogen auf den Gehalt an polymerisierbarem Material, eingesetzt werden können. Aufgrund ihrer hohen Aktivität und vorzüglichen Wasserlöslichkeit können die erfindungsgemäßen Fotoinitiatoren bevorzugt in einer Menge von 0,1-10 Gew. %, insbesondere von 0,5-5 Gew. %, bezogen auf den Gehalt an polymerisierbaren Material, den wäßrigen fotopolymerisierbaren Systemen zugesetzt werden.

Es ist vorteilhaft, neben den erfindungsgemäßen Fotoinitiatoren in den fotopolymerisierbaren Systemen Reaktionsbeschleuniger einzusetzen. Als Reaktionsbeschleuniger können beispielsweise organische Amine, Phosphine, Alkohole und/oder Thiole, die alle mindestens eine zum Heteroatom α-ständige CH-Gruppe aufweisen, zugesetzt Werden. Geeignet sind z.B. primäre, sekundäre und tertiäre aliphatische, aromatische, araliphatische oder heterozyklische Amine wie etwa Butylamin, Dibutylamin, Tributylamin, Cyclohexylamin, Benzyldimethylamin, Di-cyclohexylamin, Triethanolamin, N-Methyldiethanolamin, Phenyl-diethanolamin, Piperidin, Piperazin, Morpholin, Pyridin, Chinolin, p-Dimethylaminobenzoesäureester, 4,4'-Bis-dimethylamino-benzophenon (Michlers Keton) oder 4,4'-Bisdiethylamino-benzophenon. Besonders bevorzugt sind tertiäre Amine wie beispielsweise Triethylamin, Triisopropylamin, Tributylamin, Octyl-dimethylamin, Dodecyl-dimethylamin, Triethanolamin, N-Methyldiethanolamin, N-Butyl-diethanolamin oder Tris-(hydroxypropyl)-amin.

Diese häufig auch als Co-Initiatoren bezeichneten Reaktionsbeschleuniger werden ebenfalls in den hierfür üblichen mengen eingesetzt.

Wäßrige fotopolymerisierbare Systeme, die als Reaktionsbeschleuniger ein tertiäres organisches Amin enthalten, stellen eine besonders bevorzugte Form der vorliegenden Erfindung dar.

Durch die Einwirkung von energiereicher Strahlung, vorzugsweise UV-Licht, auf die die erfindungsgemäßen Fotoinitiatoren enthaltenden fotopolymerisierbaren Systeme kann die Fotopolymerisation ausgelöst werden. Die Fotopolymerisation erfolgt nach an sich bekannten Methoden durch Bestrahlen mit Licht oder UV-Strahlung des Wellenlängenbereichs von 250-500 nm, vorzugsweise von 300-400 nm. Als Strahlenquellen können Sonnenlicht oder künstlische Strahler verwendet werden. Vorteilhaft sind zum Beispiel Quecksilberdampf-Hochdruck-, -Mitteldruck- oder -Niederdrucklampen sowie Xenon- und Wolframlampen.

Die Durchführung der Fotopolymerisation unter Verwendung der erfindungsgemäßen Fotoinitiatoren kann sowohl diskontinuierlich als auch kontinuierlich geschehen. Die Bestrahlungsdauer hängt von der Art der Durchführung, von der Art und Konzentration der eingesetzten polymerisierbaren Materialien, von Art und Menge der verwendeten Fotoinitiatoren und von der Intensität der Lichtquelle ab und kann im Falle von massepolymerisation je nach Größe des Ansatzes im Bereich weniger Sekunden bis Minuten, bei Großansätzen etwa aber auch bis im Stundenbereich liegen.

Die Polymerisationstemperatur ist frei wählbar und kann + 5 bis ca. 100°C betragen, wobei die Durchführung bei Raumtemperatur bevorzugt ist. Eventuelle freiwerdende Polymerisationswärme kann durch übliche Kühlungsmaßnahmen abgeführt werden.

Je nach Art und Gehalt an polymerisierbarem Material in den erfindungsgemäßen wäßrigen fotopolymerisierbaren Systemen können durch Fotopolymerisation wäßrige Polymerisat-Lösungen oder Polymerisat-Dispersionen erhalten werden, deren Gehalt an Polymermaterial zwischen 10 und 80 Gew. %, üblicherweise zwischen 30 und 70 Gew. % liegt. Aufgrund der hohen Wirksamkeit der erfindungsgemäßen Fotoinitiatoren erfolgt die Umsetzung von Monomer- in Polymermaterial praktisch quantitativ. Überraschend hierbei ist, daß mit den erfindungsgemäßen Fotoinitiatoren im Vergleich zu bekannten Fotoinitiatoren bei sonst gleichen Prozeßbedingungen Polymerisate mit erheblich höheren mittleren

Molmassen erhalten werden können. Dementsprechend können die nach dem erfindungsgemäßen Verfahren hergestellten wäßrigen polymerisat-Lösungen und Polymerisat-Dispersionen in einem vielfältigen und weiten Anwendungsbereich eingesetzt werden. So eignen sich die Lösungspolymerisate beispielsweise hervorragend als Flockungsmittel für die Abwasserreinigung, als Hilfsmittel bei der Papierherstellung und in der Textilindustrie. Die Polymerisat-Dispersionen eignen sich vorzüglich zum Beschichten von Papier, Vliesstoffen und Leder und lassensich ebenfalls als Grundmaterialien für Anstriche und Klebemittel einsetzen.

Aus den nach dem erfindungsgemäßen Verfahren Hergestellten Polymerisat-Lösungen bzw. -Dispersionen läßt sich auch das Polymermaterial nach üblichen Verfahren isolieren und kann damit in reiner Form für die verschiedensten Anwendungszwecke zur Verfügung gestellt werden. So eignen sich beispielsweise hochmolekulare teilvernetzte Polymerisate auf Acrylsäurebasis aufgrund ihres hydrophilen Charakters besonders als Absorptionsmaterialien für Wasser bzw. wäßrige Elektrolytlösungen wie etwa Körperflüssigkeiten. Solche Polymermaterialien verfügen über eine hohe Kapazität und können oft ein Vielfaches ihres Eigengewichtes an derartigen Flüssigkeiten binden. Sielassen sich somit besonders vorteilhaft bei der Herstellung von Saugkörpern, etwa in Hygiene- und Körperpflegeartikeln wie beispielsweise Babywindeln verwenden. Hierbei können diese Polymerisate in loser oder kompakter Form oder aufgebracht auf geeignete Trägermaterialien, wie Textilgebilde auf Gewebe- oder Vliesbasis oder auf Papiererzeugnisse zur Anwendung gelangen.

Die nachfolgenden Beispiele beschreiben die Herstellung der Fotoinitiatoren, ihre Anwendung in wäßrigen und nichtwäßrigen Systemen und zeigen im Vergleich mit bekannten Fotoinitiatoren deren vorteilhafte Eigenschaften bei der Fotopolymerisation in wäßrigen Systemen im Hinblick auf die Erzielung einer besonders hohen Molmasse im Polymerisat.

Beispiel 1:

Herstellung von 4-(2-Hydroxyethoxy)-phenyl-2-hydroxy-2-propylketon

a) Zu 880 g (6,6 Mol) wasserfreiem Aluminiumchlorid in 480 ml Dichlormethan werden bei −5 bis 0°C 336 g (3,2 Mol) Isobuttersäurechlorid innerhalb von 40 Minuten unter Rühren zugetropft. Danach werden bei der gleichen Temperatur 540 g (3,0 Mol) 2-Phenoxyethylacetat innerhalb von 2 Stunden zugetropft. Man rührt nach der Beendigung des Zutropfens noch weitere 2 Stundenbei der angegebenen Temperatur und gießt die Reaktionsmischung dann in ein Gemisch aus 1,8 l konzentrierter Salzsäure und 5 kg Eis. Die organische Phase wird abgetrennt und die wäßrige Schicht mit Dichlormethan extrahiert. Die vereinigten organischen Phasen werden mit Wasser gewaschen, getrocknet, eingeengt und der Rückstand im Vakuum destilliert.

Man erhält 740 g (98,7 %) 4-(2-Acetoxyethoxy)phenyl-2-propylketon mit Siedepunkt 145-152 °C/0,3-0,5 Torr.

b) 250 g (1,0 Mol) 4-(2-Acetoxyethyloxy)-phenyl-2-propylketon werDen in 200 ml Eisessig gelöst und unter Rühren bei 25 °C innerhalb von 2 Stunden mit 192 g (1,2 Mol) Brom versetzt. Es wird ca. 10 Stunden nachgerührt und dann in 3 l Eisessig eingegossen. Das Produkt wird mit Essigsäureethylester extrahiert. Die vereinigten Extrakte werden getrocknet und durch Einengen erhält man 365 g eines dickflüssigen Öles. Dieses wird in 1 l Ethanol gelöst und unter Rühren bei 25 °C innerhalb von 20 Minuten mit 380 g 32 %iger Natronlauge versetzt. Es wird 10 Minuten nachgerührt und dann das Ethanol entfernt. Der ölige Rückstand wird in 3 l Eiswasser gegeben und diese mischung extrahiert man mehrmals mit insgesamt 1,5 l Essigsäureethylester. Nach dem Trocknen, Filtrieren und Einengen der Lösung werden 250 g öliges Rohprodukt isoliert. Durch Umkristallisation aus Aceton/Petrolether und/oder chromatographische Aufreinigung erhält man 145 g (65%) 4-(2-Hydroxyethoxy)-phenyl-2-hydroxy-2-propylketon in Form einer farblosen Festsubstanz vom Schmelzpunkt 88-90 °C.

Beispiel 2:

Analog zu Beispiel 1, jedoch mit 2-Phenylethylacetat als Ausgangssubstanz, wird erhalten: 4-(2-Hydroxyethyl)phenyl-2-hydroxy-2-propylketon

Beispiel 3:

Analog, jedoch mit 3-Phenylpropylacetat als Ausgangssubstanz, wird erhalten: 4-(3-Hydroxypropyl)-phenyl-2-hydroxy-2-propylketon

Beispiel 4:

Analog, jedoch mit 4-Phenylpropionsäuremethylester als Ausgangssubstanz, wird erhalten: 4-(2-Hydroxycarbonylethyl)-phenyl-2-hydroxy-2-propylketon.

Beispiel 5:

Analog, jedoch mit 4-Phenylbuttersäuremethylester als Ausgangssubstanz, wird erhalten: 4-(2-Hydroxycarbonylpropyl)-phenyl-2-hydroxy-2-propylketon vom Schmelzpunkt 57°C.

Durch Umsetzung der erhaltenen Säure mit wäßriger Natriumcarbonatlösung wird das entsprechende Natriumsalz erhalten. Schmelzpunkt: 210°.

Durch Umsetzung mit wäßriger Ammoniumcarbonatlösung wird das entsprechende Ammoniumsalz erhalten. Schmelzpunkt: 142°.

Beispiel 9

Strahlungshärtung einer nichtwäßrigen Beschichtung

Ein UV-härtbares Bindemittelsystem, das aus 75 Gew.-Teilen eines oligomeren Epoxidacrylates (Laromer® LR 8555 der Fa. BASF) und 25 Gew.-Teilen Hexandioldiacrylat besteht, wird mit 5 Gew.-Teilen 4-(2-Hydroxyethoxy)-phenyl-2-hydroxy-2-propylketon (Initiator nach Beispiel 1) versetzt.

Die gebrauchsfertige Formulierung wird auf entfettete Glasplatten (10 x 10cm) mit Spiralrakeln in einer Dicke von 50 µm aufgebracht. Anschließend werden die Beschichtungen in einem Bestrahlungsgerät ("Mini-Cure"-Gerät der Fa. Primarc Ltd.) unter einer Quecksilbermitteldrucklampe (Lampenleistung 80 Watt/cm) mit einer Bandgeschwindigkeit von 10 m/min gehärtet. Der Belichtungsabstand beträgt ca. 10cm.

Es werden voll ausgehärtete, klebfreie Beschichtungen erhalten.

In analoger Weise erhält man gleichermaßen gute Ergebnisse mit den Initiatoren der Beispiele 2-8.

Beispiel 10

Strahlungshärtung einer nichtwäßrigen Beschichtung

Ein UV-härtbares Bindemittelsystem, bestehend aus 60 Gew.-Teilen eines acrylierten Polyurethan-Prepolymers (Prepolymer VPS 1748, Fa. Degussa AG), 40 Gew.-Teilen Hexandioldiacrylat, 15 Gew.-Teilen Pentaerythrittriacrylat und 5 Gew.-Teilen 4-(2-Hydroxyethyl)-phenyl-2-hydroxy-2-propylketon (Initiator nach Beispiel 2) wird analog zu Beispiel 6 zu 50µm dicken Beschichtungen verarbeitet und bei einer Bandgeschwindigkeit von 30 m/min gehärtet. Es werden voll ausgehärtete, klebfreie Beschichtungen erhalten.

Die entsprechende Verwendung der Initiatoren nach Beispiel 1 sowie 3-8 ergibt gleichermaßen gute Ergebnisse.

Beispiel 11

UV-härtbare Druckfarbe

63,5 Teilen eines Epoxidacrylatharzes (Laromer® 8555 der Firma BASF, Ludwigshafen) werden mit 36,5 Teilen Butandioldiacrylat und 20 Teilen Heliogenblau auf einer Dreiwalze angerieben. Innerhalb von 10 minuten werden 5 Teile 4-(2-Hydroxyethyl)-phenyl-2-hydroxy-2-propylketon (Initiator nach Beispiel 2) in die Suspension eingerührt. Die so erhaltene Druckfarbe wird in 1µm Schichtdicke auf Kunstdruckpapier verdruckt und bei einer Bandgeschwindigkeit von 50 m/min mit einer Strahlungsleistung von 160 W/cm ausgehärtet. Die erhaltenen Druckbogen sind sofort stapelfähig.

Analog Beispiel 11 können die in den Beispielen 1 sowie 3-8 genannten Fotoinitiatoren als UV-Härter für Druckfarben verwendet werden.

Beispiel 12

UV-härtbarer Weiß-Lack

63,5 Gew.-Teile eines Urethanacrylat-Harzes (Uvimer® 530 der Firma Bayer, Leverkusen) werden mit 36,5 Gew.-Teilen Butandioldiacrylat und 100 Teilen Titandioxid (Anatas) in einer Porzellankugelmühle vermahlen. Anschließend werden 5 Gew.-Teile 4-(2-Hydroxyethoxy)-2-hydroxy-2-propylketon (Initiator nach Beispiel 1) und 3 Gew.-Teile N-Methyldiethanolamin eingerührt. Der in einer Schichtdicke von 10µm auf Glasplatten aufgebrachte Lack läßt sich bei einer Bandgeschwindigkeit von 50 m/min und mit einer Strahlungsleistung von 160 W/cm zu einem geruchlosen, vergilbungsfreien Film aushärten.

Analog Beispiel 12 können die in den Beispielen 2 bis 8 und 5 genannten Verbindungen als Fotoinitiatoren in einen pigmentierten Lack eingearbeitet werden.

Beispiel 13

Strahlungshärtung einer Beschichtung auf Basis eines wäßrigen Systems

20 g eines Mattierungsmittels auf Basis Kieselsäure (Mattierungsmittel OK 412 der Firma Degussa, Frankfurt/M.) Werden in 166 g einer 50 %igen wäßrigen Emulsion eines ungesättigten Acrylatharzes (Laromer® LR 8576 der Forma BASF AG; Ludwigshafen) dispergiert. Nach einer Standzeit von 18 Stunden werden weitere 166 g der 50 %igen wäßrigen Emulsion des Ungesättigten Acrylatharzes und 35,2 g Wasser unter Rühren zugegeben und 5 g der Verbindung 4-(2-Hydroxyethoxy)-phenyl-2-hydroxy-2-propylketon (Initiator und Beispiel 1) eingerührt.

Die gebrauchsfertige Formulierung wird auf entfettete Glasplatten (10 x 10 cm) mit Spiralrakeln in einer Dicke von 50 µm aufgebracht. Die beschichteten Glasplatten werden 15 Minuten bei 100°C getrocknet. Anschließend werden die Beschichtungen in einem Bestrahlungsgerät ("Mini-Cure"-Gerät der Fa. Primarc Ltd.) unter einer Quecksilbermitteldrucklampe (Lampenleistung 80 Watt/cm) mit einer Bandgeschwindigkeit von 10 m/min gehärtet. Der Belichtungsabstand beträgt ca. 10cm.

Es werden voll ausgehärtete, klebfreie Beschichtungen erhalten.

In analoger Weise erhält man gleichermaßen gute Ergebnisse mit den Initiatoren der Beispiele 2-8.

Beispiel 14

Fotopolymerisation im wäßrigen System (Verglichsversuche)

Als fotopolymerisierbares wäßriges System wird eine Lösung aus.

21 Gew. % Natriumacrylat
9 Gew. % Acrylsäure
70 Gew. % Wasser

bereitet.

In jeweils 50 g dieser Lösung werden 75 mg (0,5 Gew%, bezogen auf die Menge an polymerisierbaren Verbindungen) an Fotoinitiator zusammen mit 150 mg (1,0 Gew. %, bezogen auf die Menge an polymerisierbaren Verbindungen).

Triethanolamin als Coinitiator eigerührt. Als Fotoinitiator werden hierbei verwendet 4-(2-Hydroxyethoxy)-phenyl-2-hydroxy-2-propylketon (Initiator und Beispiel 1) und zum Vergleich die bekannten Fotoinitiatoren 1-Phenyl-2-hydroxy-2-methylpropanon-1 (Darocur® 1173, Fa. E. Merck) und 4-(Benzoylbenzyl)-trimethylammoniumchlorid (Quantacure® BTC, Fa. Ward Blenkinsop). Die erste Substanz sowie die zweite der beiden Vergleichssubstanzen lösen sich hierbei vollständig in dem wäßrigen System, die erste der Vergleichssubstanzen läßt sich nur mehr oder weniger gut dispergieren.

Die Proben werden in Küvetten gefüllt und diese jeweils exakt 30 Sekunden mit einer Hg-Lampe der Strahlungsleistung 180 W (Type Q 600, Fa. Heraeus Original Hanau) im Abstand von 5 cm bestrahlt.

Nach erfolgter Fotopolymerisation werden die Polymerisat-Lösungen mit wäßriger Natronlauge neutralisiert und durch Viskositätsmessung Die erreichten mittleren Molmassen der Polymerisate bestimmt. Diese geben Aufschluß über den erzielten Polymerisationsgrad und somit über die Effektivität des jeweils eingesetzten Fotoinitiators.

Die nachfolgende Tabelle 1 zeigt das Ergebnis

Tabelle 1

| Fotoinitiator | mittlere Molmasse des Polymerisates |
|---|---|
| 4-(2-Hydroxyethoxy)-phenyl-2-hydroxy-2-propylketon (erfindungsgemäße Verbindung) | $1{,}4 \cdot 10^6$ |
| 1-Phenyl-2-hydroxy-2-methylpropanon-(1) (Vergleichssubstanz) | $1{,}0 \cdot 10^6$ |
| 4-(Benzoylbenzyl)-trimethyl-ammoniumchlorid (Vergleichssubstanz) | $0{,}23 \cdot 10^6$ |

Es zeigt sich, daß sich bei Verwendung des erfindungsgemäßen Fotoinitiators erheblich Höhere mittlere Molmassen im Polymerisat erreichen lassen als mit den bekannten Fotoinitiatoren bei sonst gleichen Versuchsbedingungen.

**Patentansprüche**

1. Verbindungen der allgemeinen Formel I

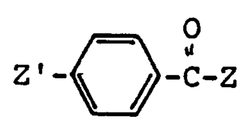

(I)

worin

Z $CR^1R^2(OR^3)$ bedeutet mit
$R^1$ H, $C_{1-6}$-Alkyl oder Phenyl,
$R^2$ H, $C_{1-6}$-Alkyl oder $C_{1-6}$-Alkoxy,
$R^3$ H, $C_{1-6}$-Alkyl oder $C_{1-6}$-Alkanoyl

und

Z' $Y-[(CH_2)_m-X]_n$-bedeutet mit
X $CH_2$ oder O,
y OH; COOH, $SO_3H$ einschließlich deren Alkali-, Erdalkali- oder Ammoniumsalze sowie deren Salze organischer Stickstoffbasen; NRR' mit R und R' jeweils H, $C_{1-20}$-Alkyl oder $C_{1-4}$-Hydroxyalkyl, gegebenenfalls quarterniert oder in Form der Säureadditionssalze, n, m jeweils die Zahlen 1-4.

2. Eine Verbindung nach Anspruch 1 aus der Gruppe
4-(2-Hydroxyethyl)-phenyl-2-hydroxy-2-propylketon
4-(3-Hydroxypropyl)-phenyl-2-hydroxy-2-propylketon
4-(2-Aminoethyl)-phenyl-2-hydroxy-2-propylketon
4-(3-Aminopropyl)-phenyl-2-hydroxy-2-propylketon
4-(2-Hydroxycarbonylethyl)-phenyl-2-hydroxy-2-propylketon
4-(3-Hydroxycarbonylpropyl)-phenyl-2-hydroxy-2-propylketon
4-(2-Hydroxyethoxy)-phenyl-2-hydroxy-2-propylketon
4-(2-Aminoethoxy)-phenyl-2-hydroxy-2-propylketon
4-[2-(Hydroxymethoxy)-ethoxy]-phenyl-2-hydroxy-2-propylketon
4-(2-Hydroxydiethoxy)-phenyl-2-hydroxy-2-propylketon
4-[2-(Hydroxycarbonylmethoxy)-ethoxy]-phenyl-2-hydroxy-2-propylketon
4-(2-Hydroxyethoxy)-diethoxyacetophenon

3. Die Verbindung 4-(2-Hydroxyethoxy)-phenyl-2-hydroxy-2-propylketon.

4. Verwendung der Verbindungen der Formel I, gegebenenfalls zusammen mit bekannten Fotoinitiatoren und/oder Sensibilisatoren, als Fotoinitiatoren für die Fotopolymerisation ethylenisch ungesättigter Verbindungen oder solche enthaltender Systeme.

5. Verwendung nach Anspruch 4 als Fotoinitiatoren für die Fotopolymerisation von ethylenisch ungesättigten Verbindungen in wäßrigen Systemen.

6. Verwendung nach Anspruch 4 zur Herstellung wäßriger Polymerisat-Lösungen oder Polymerisat-Dispersionen.

7. Verwendung nach Anspruch 4 bei der Strahlungshärtung wäßriger Präpolymerdispersionen.

8. Verfahren zur Fotopolymerisation ethylenisch ungesättigter Verbindungen oder solche enthaltender Systeme, dadurch gekennzeichnet, daß man dem zu polymerisierenden Gemisch vor der Auslösung der Fotopolymerisation mindestens eine Verbindung der Formel I als Fotoinitiator zusetzt.

9. Verfahren nach Anspruch 8, dadurch gekennzeichnet, daß man dem zu polymerisierenden Gemisch vor der Auslösung der Fotopolymerisation 0,01 bis 20 Gew.% einer Verbindung der Formel I zusetzt.

10. Fotopolymerisierbare Systeme, enthaltend mindestens eine ethylenisch ungesättigte fotopolymerisierbare Verbindung sowie gegebenenfalls weitere bekannte und übliche Zusatzstoffe, dadurch gekennzeichnet, daß sie mindestens eine Verbindung der Formel I als Fotoinitiator enthalten.

11. Fotopolymerisierbares System nach Anspruch 10, dadurch gekennzeichnet, daß es 0,01 bis 20 Gew.% eine Verbindung der Formel I enthält.

12. Wäßriges fotopolymerisierbares System enthaltend mindestens eine ethylenisch ungesättigte fotopolymerisierbare Verbindung sowie gegebenenfalls weitere bekannte und übliche Zusatzstoffe, dadurch gekennzeichnet, daß es mindestens eine Verbindung der Formel I als Fotoinitiator enthält.

13. Verfahren zur Herstellung von wäßrigen Polymerisat-Lösungen oder Polymerisat-Dispersionen durch Fotopolymerisation von in wäßrigem Medium befindlichen ethylenisch ungesättigten Verbindungen, dadurch gekennzeichnet, daß dem zu polymerisierenden Gemisch vor der Auslösung der Fotopolymerisation mindestens eine Verbindung der Formel I als Fotoinitiator zugesetzt wird.

14. Verfahren zur Herstellung wasserlöslicher oder hydrophiler Polymerisate durch Fotopolymerisation von in wäßrigem Medium befindlichen ethylenisch ungesättigten Verbindungen, dadurch gekennzeichnet, daß die Fotopolymerisation in Gegenwart von Fotoinitiatoren der Formel I durchgeführt wird.

**Revendications**

1. Composés de formule générale I

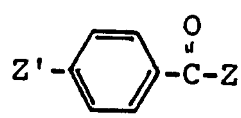

(I)

où

Z représente $CR^1R^2(OR^3)$ où
$R^1$ représente H, alcoyle en $C_{1-6}$ ou phényle,
$R^2$ représente H, alcoyle en $C_{1-6}$ ou alcoxy en $C_{1-6}$
$R^3$ représente H, alcoyle en $C_{1-6}$ ou alcanoyle en $C_{1-6}$ et
Z' représente $Y-[(CH_2)_m-X]_m$ où
X représente $CH_2$ ou O
y représente OH; COOH, $SO_3$h, y compris ses sels de métaux alcalins, alcalino-terreux ou d'ammonium ainsi que ses sels de bases azotées organiques; NRR' où R et R' représentent chacun H, alcoyle en $C_{1-20}$ ou hydroxyalcoyle en $C_{1-4}$, éventuellement quaternisé sous la forme des sels d'addition d'acides
n, m représentent à chaque fois le nombre 1-4.

2. Composés selon la revendication 1 du groupe
4-(2-hydroxyéthyl)-phényl-2-hydroxy-2-propylcétone

4-(3-hydroxypropyl)-phényl-2-hydroxy-2-propylcétone
4-(2-aminoéthyl)-phényl-2-hydroxy-2-propylcétone
4-(3-aminopropyl)-phényl-2-hydroxy-2-propylcétone
4-(2-hydroxycarbonyléthyl)-phényl-2-hydroxy-2-propylcétone
4-(3-hydroxycarbonylpropyl)-phényl-2-hydroxy-2-propylcétone
4-(2-hydroxyéthoxy)-phényl-2-hydroxy-2-propylcétone
4-(2-aminoéthoxy)-phényl-2-hydroxy-2-propylcétone
4-[2-(hydroxyméthoxy)-éthoxy]-phényl-2-hydroxy-2-propylcétone
4-(2-hydroxydiéthoxy)-phényl-2-hydroxy-2-propylcétone
4-[2-(hydroxycarbonylméthoxy)-éthoxyl-phényl-2-hydroxy-2-propylcétone
4-(2-hydroxyéthoxy)-diéthoxyacétophénone.

3. Le composé 4-(2-hydroxyéthoxy)phényl-2-hydroxy-2-propylcétone.

4. Application des composés de formule I, éventuellement avec des photoinitiateurs et/ou sensibilisateurs connus comme photosensibilisateurs pour la photopolymérisation des composés éthyléniquement insaturés ou de systèmes qui en contiennent.

5. Application selon la revendication 4, comme photoinitiateurs pour la photopolymérisation de composés éthyléniquement insaturés dans des systèmes aqueux.

6. Application selon la revendication 4, à la préparation de solutions ou de dispersions aqueuses de polymère.

7. Application selon la revendication 4, au durcissement par irradiation de dispersions aqueuses de prépolymère.

8. procédé de photopolymérisation de composés éthyléniquement insaturés ou de systèmes qui en contiennent, caractérisé en ce qu'on ajoute au mélange à polymériser avant déclenchement de la photopolymérisation au moins un composé de formule I comme photoinitiateur.

9. Procédé selon la revendication 8, caractérisé en ce qu'on ajoute au mélange à polymériser avant le déclenchement de la photopolymérisation de 0,01 à 20% en poids d'un composé de formule I.

10. Systèmes photopolymérisablés contenant au moins un composé photopolymérisable éthyléniquement insaturé ainsi qu'éventuellement d'autres additifs connus et habituels, caractérisés en ce qu'ils contiennent au moins un composé de formule I comme photoinitiateur.

11. Système photopolymérisable selon la revendication 10, caractérisé en ce qu'il contient de 0,01 à 20% en poids d'un composé de formule I.

12. Système photopolymérisable aqueux contenant au moins un composé photopolymérisable éthyléniquement insaturé ainsi qu'éventuellement d'autres additifs connus et habituels, caractérisé en ce qu'il contient au moins un composé de formule I comme photoinitiateur.

13. Procédé de préparation de solutions ou de dispersions aqueuses de polymère par photopolymérisation de composés éthyléniquement insaturés se trouvant en milieu aqueux, caractérisé en ce qu'on ajoute au mélange à polymériser au moment du déclenchement de la photopolymérisation au moins un composé de formule I comme photoinitiateur.

14. Procédé de préparation de polymères solubles dans l'eau ou hydrophiles par photopolymérisation de composés éthyléniquement insaturés se trouvant en milieu aqueux, caractérisé en ce qu'on conduit la photopolymérisation en présence de photoinitiateurs de formule I.

**Claims**

1. Compounds of the general formula I

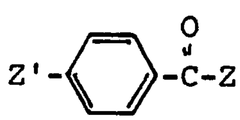

(I)

wherein

Z is $CR^1R^2(OR^3)$,
$R^1$ being H, $C_{1-6}$-alkyl or phenyl,
$R^2$ being H, $C_{1-6}$-alkyl or $C_{1-6}$-alkoxy and
$R^3$ being H, $C_{1-6}$-alkyl or $C_{1-6}$-alkanoyl and
Z' is $Y-[(CH_2)_m-X]_n-$,
X being $CH_2$ or O,
Y being OH; COOH or $SO_2H$ including the alkali metal, alkaline earth metal or ammonium salts thereof and also the salts thereof with organic nitrogen bases; or NRR' in which R and R' are in each case H, $C_{1-20}$-alkyl or $C_{1-4}$-hydroxyalkyl, if appropriate quaternized or in the form of the acid addition salts, and n and m each being the numbers 1-4.

2. A compound according to Claim 1 from the group comprising
4-(2-hydroxyethyl)-phenyl 2-hydroxy-2-propyl ketone
4-(3-hydroxypropyl)-phenyl 2-hydroxy-2-propyl ketone

4-(2-aminoethyl)-phenyl 2-hydroxy-2-propyl ketone
4-(3-aminopropyl)-phenyl 2-hydroxy-2-propyl ketone
4-(2-hydroxycarbonylethyl)-phenyl 2-hydroxy-2-propyl ketone
4-(3-hydroxycarbonylpropyl)-phenyl 2-hydroxy-2-propyl ketone
4-(2-hydroxyethoxy)-phenyl 2-hydroxy-2-propyl ketone
4-(2-aminoethoxy)-phenyl 2-hydroxy-2-propyl ketone
4-[2-(hydroxymethoxy)-ethoxy]-phenyl 2-hydroxy-2-propyl ketone
4-(2-hydroxydiethoxy)-phenyl 2-hydroxy-2-propyl ketone
4-[2-(hydroxycarbonylmethoxy)-ethoxy]-phenyl 2-hydroxy-2-propyl ketone
4-(2-hydroxyethoxy)-diethoxyacetophenone.

3. The compound 4-(2-hydroxyethoxy)-phenyl 2-hydroxy-2-propyl ketone.

4. Use of the compounds of the formula I, if appropriate together with known photoinitiators and/or sensitizers, as photoinitiators for the photopolymerization of ethylenically unsaturated compounds or systems containing these.

5. Use according to Claim 4, as photoinitiators for the photopolymerization of ethylenically unsaturated compounds in aqueous systems.

6. Use according to Claim 4, for the preparation of aqueous polymer solutions or polymer dispersions.

7. Use according to Claim 4, for the radiation-curing of aqueous prepolymer dispersions.

8. Process for the photopolymerization of ethylenically unsaturated compounds or systems containing these, characterized in that at least one compound of the formula I is added, as a photoinitiator, to the mixture to be polymerized before the photopolymerization is initiated.

9. process. according to Claim 8, characterized in that 0.01 to 20% by weight of a compound of the formula I are added to the mixture to be polymerized before the photopolymerization is initiated.

10. Photopolymerizable systems containing at least one ethylenically unsaturated photopolymerizable compound and, if appropriate, other known and customary additives, characterized in that they contain at least one compound of the formula I as a photoinitiator.

11. Photopolymerizable system according to Claim 10, characterized in that it contains 0.01 to 20% by weight of a compound of the formula I.

12. Aqueous photopolymerizable system containing at least one ethylenically unsaturated photopolymerizable compound and, if appropriate, further known and customary additives, characterized in that it contains at least one compound of the formula I as a photoinitiator.

13. Process for the preparation of aqueous polymer solutions or polymer dispersions by the photopolymerization of ethylenically unsaturated compounds present in an aqueous medium, characterized in that at least one compound of the formula I is added, as a photoinitiator, to the mixture to be polymerized before the photopolymerization is initiated.

14. Process for the preparation of water-soluble or hydrophilic polymers by the photopolymerization of ethylenically unsaturated compounds present in an aqueous medium, characterized in that the photopolymerization is carried out in the presence of photoinitiators of the formula I.